# EUROPEAN PATENT APPLICATION

(11) **EP 2 499 965 A1**
(43) Date of publication of application: **19.09.2012**
(21) Application number: 11305286.4
(22) Date of filing: 15.03.2011
(51) Int. Cl.: A61B 5/00, A61B 5/11, G06F 3/01, G02B 27/01

(54) **Method of providing a person with spatial orientation information**

(71) Applicant: UNIVERSITE PARIS-SUD (PARIS 11), 91405 Orsay (FR); ESSILOR INTERNATIONAL (Compagnie Générale d'Optique), 94220 Charenton le Pont (FR)
(72) Inventor: Isableu, Brice, 91400, Orsay (FR); Giraudet, Guillaume, 94220, CHARENTON-LE-PONT (FR); Rousseau, Benjamin, 94220 CHARENTON-LE-PONT (FR)
(74) Representative: Cabinet Plasseraud

(57) **Abstract**

Method for providing a person (1) with information about his/her spatial orientation relative to a place with a vertical direction (3) defined by gravity, comprising the steps of: a) measuring a value of at least one orientation parameter (8b, 8c) of a part of the person's body with respect to the vertical direction; and b) providing the person with a visual indication of the vertical direction using the value measured in step a).

## Description

The invention relates to a method of providing spatial orientation information to a person.

The sense of spatial orientation is the ability to maintain the body orientation and/or posture in relation with environment (physical space) at rest and during motion. It relies for example on the use of visual, vestibular (organs of equilibrium located in the inner ear), proprioceptive (receptors located in the skin, muscles, tendons, and joints), auditory, sensory information.

It thus relates to the capability to assess the physical relationship between the body and the environment, and to deal with modifications in this relationship during movement.

Due to age or a given pathology (such as vestibular problems, Parkinson's disease), it may happen that some people experience a decrease in this capability, leading to difficulties in controlling their movements and balance. Also people suffering from agoraphobia may find the visual signals of a place that they receive insufficient to feel comfortable.

It is an object of the invention to provide a method for giving a person information about his or her spatial orientation, in particular information that can be easily processed by the brain to reinforce the sense of spatial orientation.

The previously mentioned object is fulfilled by a method for providing a person with information about his/her spatial orientation relative to a place with a vertical direction defined by gravity, comprising the steps of:
a) measuring a value of at least one orientation parameter of a part of the person's body with respect to the vertical direction; and
b) providing the person with a visual indication of the vertical direction using the value measured in step a).

The vertical direction is not a given vertical line but a set of lines. The direction is given by gravity in the considered place where the person is.

The "orientation parameter" measured in step a) may be any geometrical parameter defining the orientation of a part of the person's body. To fully determine the orientation of an object, three coordinates are used. In solids mechanics, the Euler angles are often used. For plane navigation, three other angles are frequently used: the yaw (where the plane is going to), the pitch (is the plane going up or down?) and the roll (is the plane turning on its right or on its left side?).

To fully determine the position of an object versus the vertical direction, only two parameters are needed, for example the pitch and the roll. They are the only ones that passengers in a plane can actually feel, because they affect their balance.

In step a), a value of at least one of these orientation parameters versus the vertical direction, for example the pitch, is measured. The value providing the orientation of a part of the body versus the vertical direction also provides the orientation of the vertical direction versus the person.

In step b), the person is given a visual indication of the vertical direction using the value measured in step a). The visual indication can be any type of indication from which the person can deduce something about how the considered body part is oriented versus the vertical direction. The visual indication can be for example alphanumerical (e.g. a written angle), and/or a drawing, and/or an image.

According to an embodiment steps a) and b) are carried out on a real time basis, so that the visual indication can be corrected at a pace compatible with the type of movement for which the person requires help.

There is an advantage in keeping the visual indication of step b) rather simple. Since it is recalculated using measurements, the calculation requirements, such as the memory and time needed, can be minimized when the definition of the visual indication based on the value measured in step a) does not require a lot of calculation.

The part of the body of which an orientation parameter is measured depends on the type of movement or balance which the person needs help for.

According to the present invention, the person is provided with information about the vertical direction on top of other pieces of information available. The visual indication provided in step b) can be considered as additional data, in order to enhance the person's sense of spatial orientation.

Also, if the person is prevented from receiving natural visual indications playing a role in spatial orientation (for example because of fog or smoke preventing from seeing surroundings), the visual indication of step b) may be the only one that the person receives and on which he or she can rely. Then the visual indication of step b) acts as a substitute.

According to embodiments of the present invention, the following features may be added and/or combined:
- the visual indication is provided by sending light beams into at least one eye of the person for creating an image of an object on the retina, the object having a property defined by a calculation using the value measured in step a) and perceivable by the person in the image as an indication of the vertical direction. The considered property of the object is a property that is naturally associated with verticality, so that the person may integrate the visual indication of step b) effortlessly. For example, if the object is a tree, the light beams are configured to obtain an image of the tree on the retina such that the tree is perceived as vertical. This is obtained by calculating the position of the tree taking into account the orientation of the person as measured in step a). The image of the object is preferably formed on the peripheral region of the retina, since that region is favoured by the brain to obtain spatial orientation data. By doing so, the person can still use his or her eyes with a minimal disturbance due to the object, the centre of his or her vision being not modified by the object.
- the object comprises a segment and the property defined by calculation includes an orientation parameter of the segment with respect to the part of the person's body, so that the segment is perceived by the person as parallel to the vertical direction. A segment kept vertical by calculation is a very efficient element to convey a notion of verticality to the person. In order to further reinforce the perception of verticality, the segment can be completed with a flat surface kept horizontal by calculation using the measured parameter(s).
- the object comprises a polyhedron or a rectangular parallelepiped. Such objects have vertical segments. They can also have horizontal surfaces. It is also easy to make sure that their image remains mostly in the peripheral region of the retina. They can easily surround the rest of what the person is looking at. According to an embodiment, the size and/or initial position of the object is selected by the person. According to a particular embodiment, the front of the rectangular parallelepiped is seen with an angle of 15° to 25°, preferably around 20°, and its rear is seen with an angle of about 5° to 15°, preferably around 10°.
- the light beams are sent from a see-through system. Thanks to the see-through system, the ability of the person to see is not decreased by the visual indication of step b), which can act as additional information regarding the spatial orientation of the person.
- the light beams are sent into both eyes of the person to create a left image and a right image of the object allowing a stereoscopic perception of the object by the person. The two images are slightly different to take into account the parallax effect. As for any other image, of the person integrates the parallax effect to allow a stereoscopic perception. This adds a notion of depth to the visual indication of step b). Thus the visual indication can be more easily processed by the person. If the visual indication is the image of an object, the object will seem more real. According to an embodiment, the parallax of the object can be adjusted by the person.
- the at least one orientation parameter includes the pitch and/or the roll of the part of the person's body. Providing a visual indication taking into account the pitch provides the person with a notion of whether his or her body part is bent forward or backward versus the vertical direction. The roll provides the person with a notion of whether he or she is bent on the right or left.
- said part is the person's head or trunk. A visual indication of the head or trunk's orientation enables the person to better control his or her global balance.
- steps a) and b) are iterated at a frequency above or equal to 24 Hz. This allows a continuous perception of the visual indication of step b), as if a real object was seen.

The invention also deals with an apparatus for providing a person with information about his/her spatial orientation relative to a place with a vertical direction defined by gravity, comprising:
- a sensor for measuring a value of at least one orientation parameter of a part of the person's body with respect to the vertical direction;
- a light source for sending the person a visual signal; and
- a calculator connected to the sensor for receiving the value and to the light source for sending data calculated using the value and defining the visual signal, the visual signal providing an indication of the vertical direction.

The sensor may comprise a gyroscope. The measured value is used by the calculator to define the visual signal. The visual signal is created by the light source based on the calculation and is guided towards at least one eye of the person.

According to embodiments of the present invention, the following features may be added and/or combined:
- the light source comprises a see-through system for sending light beams into at least one eye of the person to create an image of an object on the retina.
- the see-through system is binocular to allow a stereoscopic perception of the object by the person.
- the sensor is configured so as to measure the pitch and/or the roll of the part of the person's body.

The invention also deals with computer program product comprising one or more stored sequences of instructions that are accessible to a processor and which, when executed by the processor, causes the processor to carry out the steps of the method described here above.

The invention is about a computer-readable medium carrying one or more sequences of instructions of the above mentioned computer program product.

Non limiting embodiments of the invention will now be described with reference to the accompanying drawings, wherein:
- figure 1 is a scheme of part of an apparatus according to the present invention, worn by a person needing help with his or her spatial orientation ;
- figure 2 represents what is seen by the person through the apparatus of fig. 1, the person and his head being in a vertical position ;
- figures 3a and 3b illustrate what the person sees through the apparatus after bending his head on his right side from the vertical position of fig. 2 ;
- figures 4a and 4b illustrate what the person sees through the apparatus after turning his head left starting from the vertical position of fig. 2 ;
- figure 5a and 5b illustrate what the person sees through the apparatus after bending his head forward, towards the ground, starting from the vertical position of fig. 2 ;
- figure 6 represents the external shape of an apparatus according to the invention ;
- figure 7 illustrates an example of how the orientation of the person's head can be measured using the yaw-pitch-roll coordinates system.

Skilled artisans will appreciate that elements in the figures are illustrated for simplicity and clarity and have not necessarily been drawn to scale. For example, the dimensions of some of the elements in the figures may be exaggerated relative to other elements to improve the understanding of the embodiments of the present invention. Also, the same numeral references in the different figures correspond to a same element.

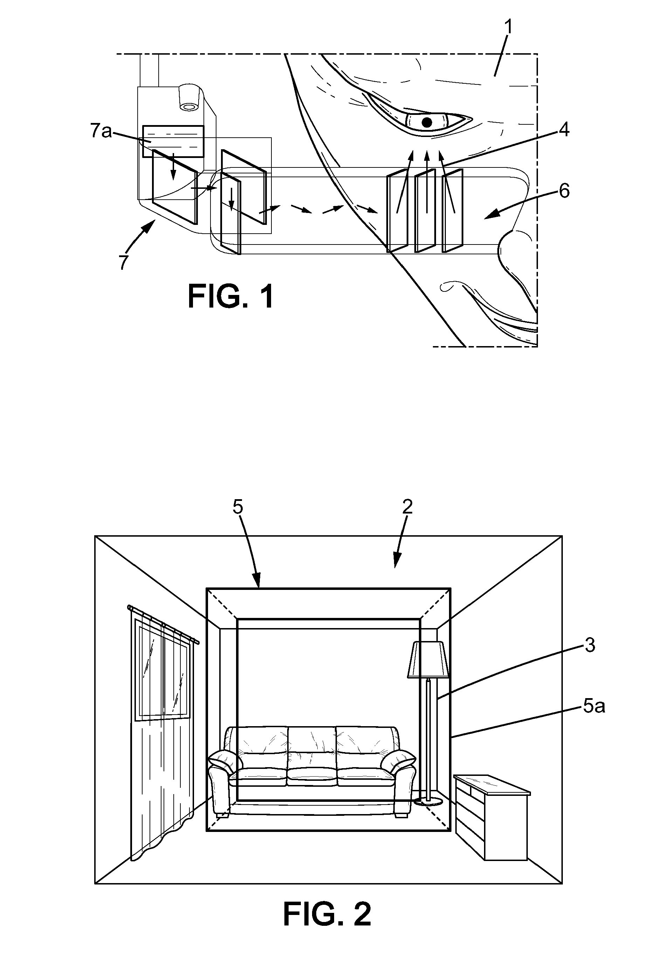

Figure 1 shows part of a person's head 1. This person needs help for his or her spatial orientation. The person is equipped with an apparatus 7 to provide this help. The apparatus comprises a light source 7a including a system of optical guides located in a see-through system 6. The light emitted by the source is guided towards at least on eye of the person in the form of light beams 4. The light beams provide visual information to the person on top of what he can normally see. This is preferably done in each eye in order to achieve a stereoscopic perception.

Documents WO03058320 and WO2004IL00813 describe examples of such optical guides that can be used in an apparatus according to the invention.

The visual indication sent to the person pertains to the vertical direction of the place where the person stands. The orientation of the person's body part is measured. In the example, the body part is the person's head. Its orientation can be measured using a gyroscope located near the light source 7a (not represented) and tight to the apparatus, the apparatus following the person's head in its movements.

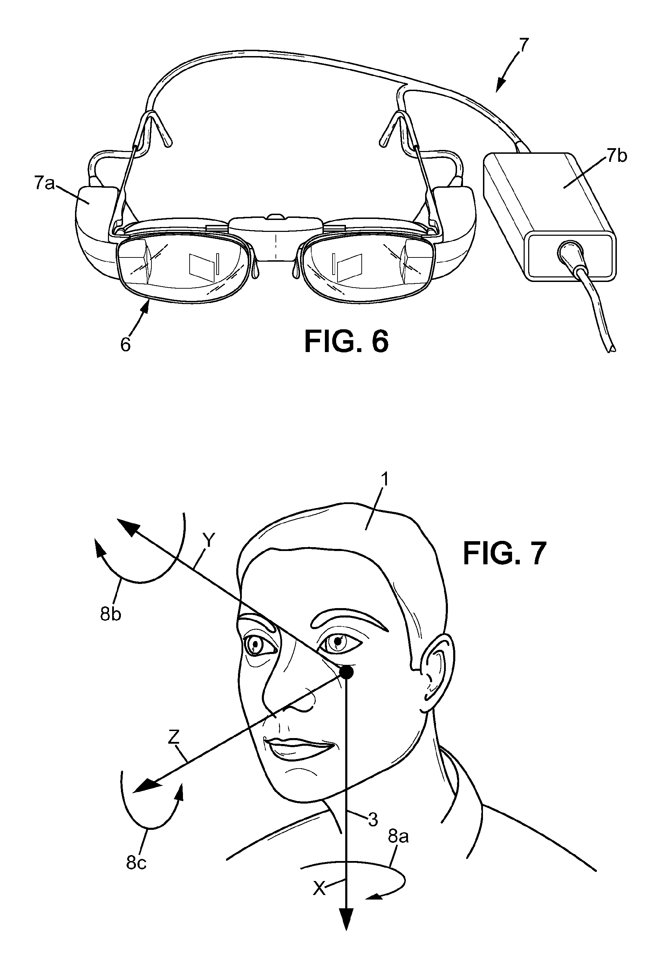

At least one orientation parameter is measured. The measured value is processed by a calculator 7b (not represented in fig. 1 but present in fig. 6). The calculator sends data to the light source in order to update the light signals emitted by the source and guided towards the eye of the person. An update is performed when the orientation of the person's head has changed versus the vertical direction.

Examples of light sources connected to an electronic calculator are given in documents WO2008062129 and W02007125257. The see-through system 6 can be connected to this kind of device. The visual indication is then corrected based on the measured value, so that the light beams are still perceived by the person as indicating the vertical direction. The correction is preferably performed on a real time basis. The visual indication is preferably updated at a frequency above or equal to 24 Hz.

Fig. 6 shows an apparatus according to the invention. The see-through system 6 can be located in glasses worn by the person, each glass having its own light source 7a for sending light beams into both eyes of the person. The calculator 7b can be located in a separate box which can also hold a battery for powering the calculator and the light sources.

Figure 7 shows an example of how the orientation of the person's head can be measured using a gyroscope, or several gyroscopes. The referential XYZ is fixed with respect to the head, with its origin somewhere close to the centre of the person's head. When the head is in a vertical position, the X axis is vertical and oriented downwards. The Z axis is horizontal, pointing in the front direction. The Y axis is perpendicular to the X and Z axis and points towards the person's right side. Then the position of the person's head can be defined by the rotation angle around the X axis, called "yaw" in navigation. After this rotation, the Y and Z axis are still horizontal. A second rotation around the Y axis defines the "pitch" angle. The pitch is positive if the person looks upwards and negative if the person looks downwards. After this second rotation, the Z axis is not horizontal any more. A third rotation around the Z axis defines the "roll" parameter, that is to say whether the person's head is bent on his right or left side.

The yaw-pitch-roll system is a convenient coordinates system. There are many others that can be used, such as Euler's one.

According to a particular embodiment of the invention, the yaw may be measured, but not used to calculate or update the visual indication sent to the person, as a modification of the yaw does not change the perception of verticality by the person. This can be understood by imagining the person standing on board a plane. When the plane's pitch or roll changes, even very slowly, the person feels it and has to adapt his or her position in order not to fall. If only the yaw changes, no such adaptation is needed.

Figure 2 shows what the person may see when equipped with an apparatus according to the invention. According to a particular embodiment, the apparatus comprises a see-through system, so that the person may see the place 2 where he or she is in. In the example the place has a vertical direction which can be materialized for example by a line 3 between two walls. The visual indication given by the light beams is perceived as an object 5 superimposed to what the person normally sees, in front of the person in the direction of Z axis as defined in fig. 7. In the example, since the person receives two images of the object, one on the retina of each eye, the object is perceived in perspective, which reinforces the impression that the object is real.

In the example, the object 5 is a rectangular parallelepiped. It comprises a first square at the front and a second one at the rear. According to a particular embodiment, the front of the rectangular parallelepiped is seen with an angle of 15° to 25°, preferably around 20°, and its rear is seen with an angle of about 5° to 15°, preferably around 10°. The object has a property that is perceived by the person as indicating the vertical direction. In the example, the vertical sides of the squares, such as the 5a one, play that role. In addition, the basis of the parallelepiped also indicates the vertical direction. The position of the object is updated using the orientation parameter measured during step a) of the process according to the invention. Using it, the position of the object is recalculated so that the side 5a still indicates the vertical direction. If the person moves his or her head, the object is moved with an opposite movement, so that line 5a remains parallel to the vertical direction, at least in the person's perception.

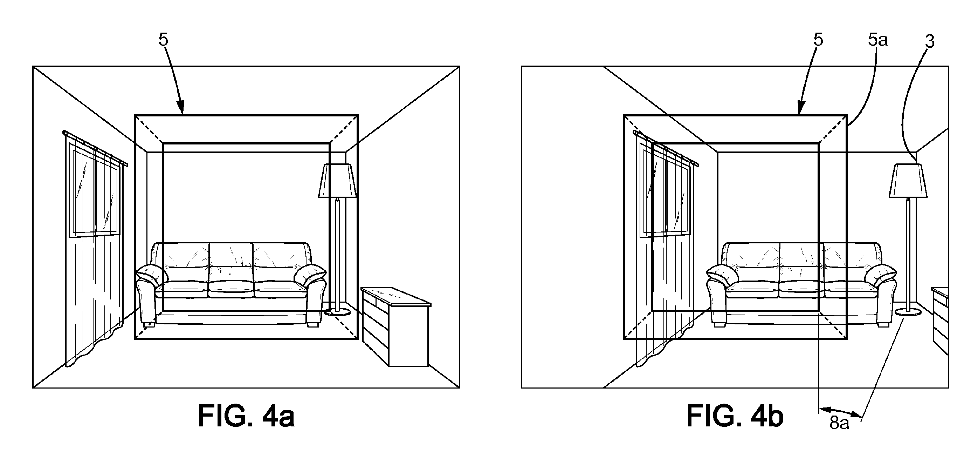

According to a particular embodiment, not all movements of the considered body part are compensated. If there is a change in the head's yaw (as defined in fig. 7), the object is not moved, so that it stays in the direction of the Z axis. This is illustrated in figures 4a and 4b. On the left image is shown what the person sees when looking straight ahead. On the right image is shown what he or she sees after turning his or her head on the left with a yaw materialised by the angle 8a. The object 5 has not been modified though the head yaw has changed. Its side 5a still indicates the vertical direction 3.

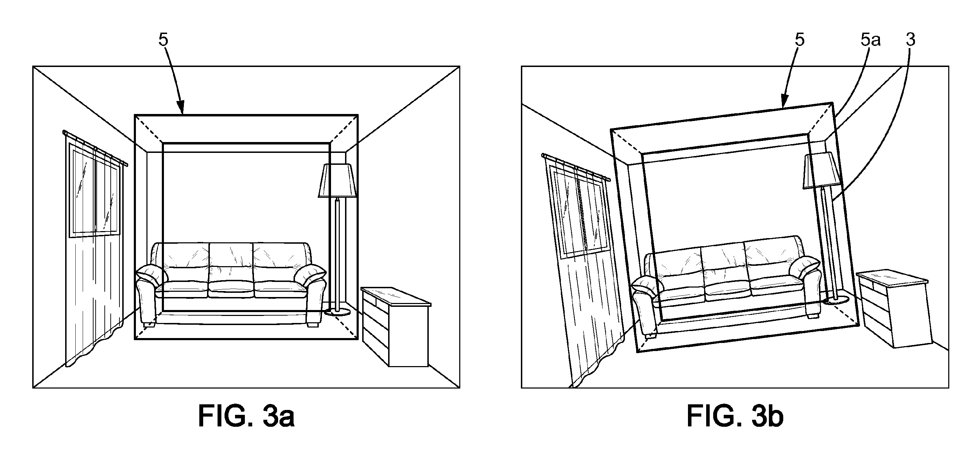

Figures 3a and 3b illustrate what happens in case the person bents his or her head on the right, that is to say with a positive roll angle according to the definition of fig. 7, the yaw and the pitch remaining the same. As a result, on the right image, the person sees the same scene with an angle. The object 5 has been rotated according to an opposite roll angle, so that its side 5a is still parallel to the vertical direction 3.

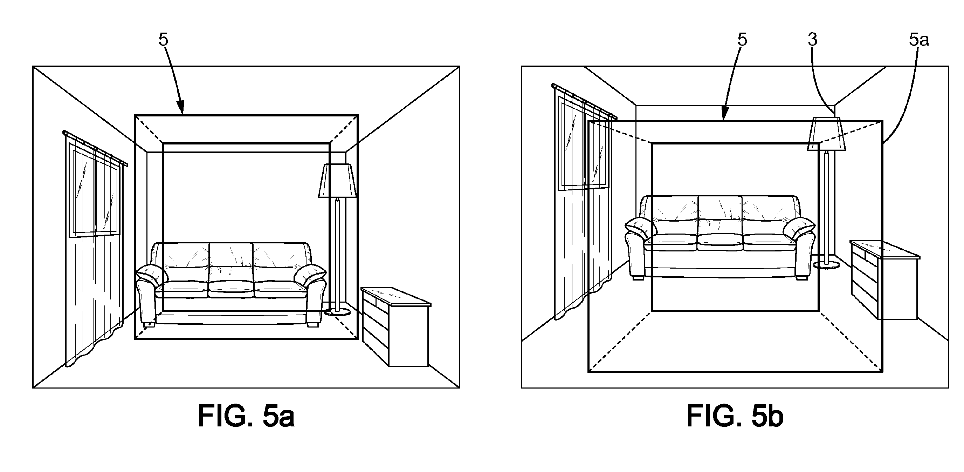

Figures 5a and 5b illustrate what happens when the person bents his or her head forward, looking towards the ground. As a result the head has a negative pitch angle, the yaw and roll remaining unchanged. In that case the object is also turned according to the opposite pitch angle, so that its side 5a is still perceived as parallel to the vertical direction 3 of the place.

Using a parallelepiped as the visual indication sent to the person has several advantages. Thanks to its shape, it remains in a peripheral region of the retina, which is where the person tends to look for spatial orientation data. Moreover, as the object remains peripheral, it normally does not affect the centre of what the person sees.

Other shapes could be used as alternatives, for instance a polyhedron or a simple vertical line.

According to particular embodiments, the person may predefine the shape of the object, its size and the parallax under which it is seen. The frequency of the position updates can also be adjusted. Thresholds can be defined for triggering an update, for instance an absolute value of pitch and/or roll, and/or of their first and/or second derivatives.

The way the apparatus is used is as follows. On a real time basis the yaw, pitch and roll of the person's head are measured by a gyroscope located near the light source 7a. The measured values are transmitted to calculator 7b. The latter calculates how to modify the object 5 so that it is still perceived by the person as indicating the vertical direction 3 of the place. To that end, the calculator sends data to the light source to adapt the light emitted and guided via the see-through system 6 towards the person's eyes. Measured modifications of the head pitch or roll result in the opposite modification of the object pitch or roll, so that the object is perceived as invariably indicating the vertical direction. This visual indication sent to the person provided a complement or a substitute (depending on whether the person can see the surroundings) helping his or her sense of spatial orientation.

## Claims

1. Method for providing a person (1) with information about his/her spatial orientation relative to a place with a vertical direction (3) defined by gravity, comprising the steps of:
a) measuring a value of at least one orientation parameter (8b, 8c) of a part of the person's body with respect to the vertical direction; and
b) providing the person with a visual indication of the vertical direction using the value measured in step a).

2. Method according to claim 1, wherein the visual indication is provided by sending light beams (4) into at least one eye of the person for creating an image of an object (5) on the retina, the object having a property defined by a calculation using the value measured in step a) and perceivable by the person in the image as an indication of the vertical direction.

3. Method according to claim 2, wherein the object comprises a segment (5a) and the property defined by calculation includes an orientation parameter of the segment with respect to the part of the person's body, so that the segment is perceived by the person as parallel to the vertical direction.

4. Method according to any of claims 2 or 3, wherein the object comprises a polyhedron or a rectangular parallelepiped.

5. Method according to any of claims 2 to 4, wherein the light beams are sent from a see-through system (6).

6. Method according to any of claims 2 to 5, wherein the light beams are sent into both eyes of the person to create a left image and a right image of the object allowing a stereoscopic perception of the object by the person.

7. Method according to any of claims 1 to 6, wherein the at least one orientation parameter includes the pitch and/or the roll of the part of the person's body.

8. Method according to any of claims 1 to 7, wherein said part is the person's head or trunk.

9. Method according to any of claims 1 to 8, wherein steps a) and b) are iterated at a frequency above or equal to 24 Hz.

10. Apparatus (7) for providing a person (1) with information about his/her spatial orientation relative to a place with a vertical direction (3) defined by gravity, comprising:
- a sensor for measuring a value of at least one orientation parameter of a part of the person's body with respect to the vertical direction;
- a light source (7a) for sending the person a visual signal; and
- a calculator (7b) connected to the sensor for receiving the value and to the light source for sending data calculated using the value and defining the visual signal, the visual signal providing an indication of the vertical direction.

11. Apparatus according to claim 10, wherein the light source comprises a see-through system (6) for sending light beams (4) into at least one eye of the person to create an image of an object (5) on the retina.

12. Apparatus according to claim 11, wherein the see-through system is binocular to allow a stereoscopic perception of the object by the person.

13. Apparatus according to any of claims 9 to 12, wherein the sensor is configured so as to measure the pitch (8b) and/or the roll (8c) of the part of the person's body.

14. A computer program product comprising one or more stored sequences of instructions that are accessible to a processor and which, when executed by the processor, causes the processor to carry out the steps of any one of claims 1 to 9.

15. A computer-readable medium carrying one or more sequences of instructions of the computer program product of preceding claim.
